# EUROPEAN PATENT APPLICATION

(11) **EP 2 018 850 A1**
(43) Date of publication of application: **28.01.2009**
(21) Application number: 07014500.8
(22) Date of filing: 24.07.2007
(51) Int. Cl.: A61K 9/16, A61K 9/20

(54) **Pharmaceutical composition comprising solifenacin or a pharmaceutically acceptable salt thereof**

(71) Applicant: Ratiopharm GmbH, 89079 Ulm (DE)
(72) Inventor: Alles, Rainer, 81476 München (DE); Kotschenreuther, Dunja, 4102 Binningen (CH); Koellner, Gertraud, 89233 Neu-Ulm (DE)
(74) Representative: Teipel, Stephan

(57) **Abstract**

The present invention relates to a pharmaceutical composition comprising solifenacin or a pharmaceutically acceptable salt thereof and a process for the preparation of said pharmaceutical composition.

## Description

The present invention relates to a pharmaceutical composition comprising solifenacin or a pharmaceutically acceptable salt thereof and a process for the preparation of said pharmaceutical composition.

Solifenacin, having the chemical name (1R,3'R)-3'-quinuclidinyl 1-phenyl-1,2,3,4-tetrahydro-2-isoquinoline carboxylate, is represented by the formula (I) below:

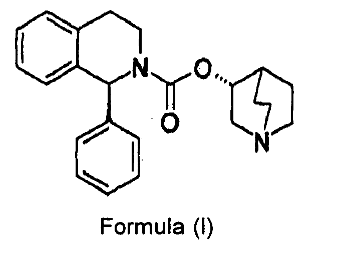

Solifenacin or salts thereof are reported to have an excellent selective antagonistic action against muscarine M₃ receptors and are useful as prophylactic or therapeutic agents of urinary diseases such as nervous pollakiuria, neurogenic bladder, nocturnal enuresis, unstable bladder, bladder contracture, and chronic cystitis as well as respiratory diseases such as chronic occlusive lung diseases, chronic bronchitis, asthma and rhinitis. Synthesis of a series of quinuclidine derivatives including solifenacin or salts thereof is disclosed in EP 801 067.

EP 1 728 791 discloses compositions of solifenacin or a salt thereof for the use in solid formulation, whereby the composition contains the crystal form of solifenacin or a salt thereof and an amorphous content within a range with no influence on the stability of the resulting product, as well as a pharmaceutical composition containing solifenacin or a salt thereof and an inhibitor of amorphous preparation.

As it is also disclosed in EP 1 728 791, it was known in the art that amorphous solifenacin succinate generated during a manufacturing process of said drug product caused destabilisation of the active pharmaceutical ingredient over time, such as degradation of the active pharmaceutical ingredient. Therefore, it was suggested in EP 1 728 791 to keep the ratio of amorphous solifenacin to crystalline solifenacin at a given value or lower, e.g. by addition of appropriate inhibitors preventing amorphisation.

W02006/070735 discloses a stable granular pharmaceutical composition which contains solifenacin or a salt thereof and a binding agent which has a stabilizing action on the solifenacin or salt thereof, and which has in particular the effect of inhibiting retention of the amorphous state of the solifenacin. To obtain a pharmaceutical composition comprising crystalline solifenacin which stays in crystalline form, a solution of the solifenacin and a binder is sprayed onto nuclei.

Therefore, it is known in the art that the higher the content of amorphous solifenacin is in a pharmaceutical composition comprising solifenacin as active ingredient, the more instable the composition becomes. Further, in particular compositions obtained by wet granulation are unstable, if they are not stabilized by an appropriate excipient acting as stabilizer.

Amorphous solifenacin exhibits a glass transition temperature range of 40°C to 60°C. Further, it is known that pure amorphous solifenacin is typically difficult if not impossible to handle as it is extremely sticky. The crystalline form of solifenacin which is used instead, however, shows the above described conversion into amorphous form. This conversion also seems to favour chemical degradation of the drug. The amorphous form on the other hand would be very desirable due to a higher bioavailability.

Therefore, the problem of the present invention is to provide a stable pharmaceutical composition comprising solifenacin wherein the solifenacin is in amorphous form.

It has now surprisingly been found that solifenacin or pharmaceutically acceptable salts thereof can be obtained in a completely amorphous, powdery form which can also be excellently handled when the amorphous form is appropriately stabilized, in particular if it is produced by spray drying, melt extrusion or grinding the solifenacin or the pharmaceutically acceptable salt thereof with a suitable excipient.

The present invention therefore relates to a pharmaceutical composition comprising solifenacin or a pharmaceutically acceptable salt thereof wherein the solifenacin is present substantially in amorphous form, the composition comprises a stabilizer and the ratio of the stabilizer to the amorphous solifenacin based on parts by weight is at least about 2:1 in the composition.

The pharmaceutical composition of the present application generally comprises solifenacin or a pharmaceutically acceptable salt thereof. The salts of solifenacin which can be used in the compositions of the present application include the acid adduct salts with mineral acids such as hydrobromic acid, hydroiodic acid, sulfuric acid, nitric acid and phosphoric acid or organic acids such as formic acid, butyric acid, propionic acid, oxalic acid, malonic acid, fumaric acid, maleic acid, lactic acid, malic acid, citric acid, tartaric acid, carbonic acid, picric acid, metansulfonic acid, glutamic acid, and succinic acid, the latter being preferred. The solifenacin is present in the pharmaceutical compositions of the present application substantially in amorphous form, such that more than about 95 wt %, more preferred more than about 99 wt %, in particular about 100 wt % of the solifenacin is present in amorphous form, based on the total weight of the composition. Therefore, most preferably no crystalline solifenacin or the pharmaceutically acceptable salt thereof can be detected within the pharmaceutical composition of the present application by any detection method known in the art, such as infrared spectroscopy, raman spectroscopy, differential scanning calorimetry (DSC) or measurement of the powder X-ray diffraction pattern, the latter being preferred. Therefore, as used herein, the term "amorphous" describes a solid devoid of long-range crystalline order. In particular, such amorphous form of a solid can be identified by the lack of discrete peaks within an X-ray powder diffraction pattern of said solid and/or observation of a glass transition point within a DSC diagram.

The term "solifenacin" as used herein also comprises pharmaceutically acceptable salts of solifenacin.

In the pharmaceutical composition of the present invention the ratio of the stabilizer to amorphous solifenacin or the pharmaceutically acceptable salt thereof based on parts per weight is at least about 2:1, preferably about 5:1 or higher, more preferably about 10:1, or higher, in particular about 20:1, or higher. More preferably the ratio of the stabilizer to amorphous solifenacin or a pharmaceutically acceptable salt thereof based on parts per weight is about 2:1 to about 20:1, in particular about 5:1 to about 10:1.

In particular, it has been found that by increasing the ratio of the stabilizer to the amorphous solifenacin in a pharmaceutical composition, a stabilization of the amorphous solifenacin can be achieved, in particular such stabilization that the amorphous solifenacin stays substantially in amorphous form. The amorphous solifenacin within the pharmaceutical composition of the present application is therefore preferably stabilized by a stabilizer, i.e. a stabilized amorphous form, in particular to keep the amorphous solifenacin in a powdery form which is easy to handle and which stabilization maintains the amorphous form stable against crystallisation particularly as long as kept dry.

Preferably the solifenacin is stabilized within the pharmaceutical composition of the present invention by covering the solifenacin with the stabilizer, and therefore the pharmaceutical composition preferably comprises solifenacin, which is covered by the stabilizer. The term "covered" is used within the present application to indicate that the amorphous solifenacin particles are at least partially, preferably totally coated or surrounded, respectively, with the stabilizer, whereby the solifenacin or a pharmaceutically acceptable salt thereof and the stabilizer are in intense, deep contact with each other. An intense, deep contact is preferably such intense and deep that the glass transition temperature of the solifenacin or a pharmaceutically acceptable salt thereof is increased compared to the untreated solifenacin or a pharmaceutically acceptable salt thereof, whereby the increase is dependent on the kind of stabilizer used. Preferably, the amorphous solifenacin particles are covered by the stabilizer in such extend that at least about 50 %, more preferred at least about 80 %, in particular at least about 95 %, e.g. about 100 % of the surface of the particles of amorphous solifenacin are covered by the stabilizer. Such a mixture is for example different to a known mixture, where the carrier and the active ingredient are coprecipitated, e.g. in roughly equal amount, and therefore also separate particles of carrier and active ingredient can be found.

Therefore, to obtain the mixture of the amorphous solifenacin or the pharmaceutically acceptable salt thereof and the stabilizer wherein the amorphous solifenacin or a pharmaceutically acceptable salt thereof is preferably at least partially covered by the stabilizer, the ratio of stabilizer to amorphous solifenacin or a pharmaceutically acceptable salt thereof based on parts per weight has to be increased, i.e. to at least 2:1, to provide enough stabilizer material, preferably to at least partially, more preferably totally cover the amorphous solifenacin particles.

Preferably the stabilized solifenacin, which is more preferably solifenacin covered by the stabilizer, is characterized by showing an increase in the glass transition temperature of about 30°C to about 60°C.

The amorphous solifenacin, which is preferably stabilized by a stabilizer, more preferably covered by a stabilizer, is preferably obtained by spray drying, in particular of a solution or dispersion of solifenacin and a stabilizer, or by melt extrusion, in particular of a mixture of solifenacin and a stabilizer, or by grinding, in particular of a mixture of solifenacin and a stabilizer, more preferably by melt extrusion, in particular of a mixture of solifenacin and a stabilizer, or grinding, in particular of a mixture of solifenacin and a stabilizer, and most preferred by melt extrusion, in particular of a mixture of solifenacin and a stabilizer.

As stabilizer to be used in the pharmaceutical compositions of the present application each pharmaceutically acceptable excipient and adjuvant can be used which can provide the stabilization or amorphous solifenacin, which preferably can provide the above described intense and deep mixture with the amorphous solifenacin and which more preferably can cover the amorphous solifenacin particles. Preferably, the stabilizers to be used in the pharmaceutical compositions of the present application are polyethylene glycol (PEG), hydroxypropyl methyl cellulose (HPMC), methyl cellulose (MC), polymethacrylate, polyvinyl pyrrolidone (PVP), polyvinyl acetate (PVAc), PVP-vinylacetate-copolymer (PVP-VA), Kollidon VA 64 (a vinylpyrrolidone-vinyl acetate copolymer), Eudragit E (a basic butylmethacrylat copolymer), lactose as well as sorbitol, mannitol, maltitol and Isomalt (artificial sugar substitute of type of sugar alcohol; nearly equimolar composition of 6-O-α-D-glucopyranosido-D-sorbitol (1,6-GPS) and 1-O-α-D-glucopyranosido-D-mannitol (1,1-GPM); e.g. obtainable as PALATINIT^{®}). If the stabilized amorphous solifenacin or a pharmaceutically acceptable salt thereof, which preferably is the amorphous solifenacin or a pharmaceutically acceptable salt thereof covered by a stabilizer, is obtained by spray drying, the use of PEG, HPMC, methylcellulose (MC), PVP-VA, PVP, maltitol and/or Isomalt is preferred, in particular PVP-VA, maltitol and/or MC. When using melt extrusion to obtain the stabilized amorphous solifenacin or the pharmaceutically acceptable salt thereof, the use of PVP-VA, mannitol, Isomalt, Eudragit E Kollidon VA 64 and/or polymethacrylate as stabilizer is preferred. When grinding is used to obtain the stabilized amorphous solifenacin or the pharmaceutically acceptable salt thereof, PVP-VA, MC and/or mannitol are preferably used as stabilizer.

In one embodiment of the present application, the stabilized amorphous solifenacin or a pharmaceutically acceptable salt thereof is preferably not obtained by spray drying, in particular spray drying of the solifenacin or the pharmaceutically acceptable salt thereof using PEG, HPMC and/or PVP as stabilizers.

To obtain the stabilized amorphous solifenacin or the pharmaceutically acceptable salt thereof by spray drying, the solifenacin or the pharmaceutically acceptable salt thereof and the stabilizer are solved or dispersed in a suitable solvent, preferably an aqueous solvent, in particular water and the resulting solution or dispersion is sprayed into a hot stream of gas, in particular air, preferably within an appropriate device, such as a spray tower and thereby very fine solution drops are obtained and the solvent is evaporated, resulting in a coprecipitate, which can be collected by an appropriate device, such as a cyclone. Preferably, the solution is pumped by a peristaltic pump in an amount of 2 - 20 g/min, preferably 3 - 10 g/min, such as 5 - 7 g/min into the spray dryer, e.g. a Büchi B-191, preferably at an air temperature of about 130°C and a resulting exhaust air temperature of 65 - 75°C and an amount of drying air of about 700 l/h.

If melt extrusion is used as method to obtain the stabilized amorphous solifenacin, the stabilizer, preferably PVP-VA, polymethacrylat or mannitol, and the solifenacin are mixed in a ratio as described above, preferably about 5:1 parts per weight to about 10:1 parts per weight, or higher, in a suitable mixer, such as a drum mixer, followed by dosing the mixture into a suitable extruder, such as a screw extruder, preferably a double screw extruder, preferably at a rate of about 1 kg/hour. The suitable extruder preferably consists of individually heatable cylinders, which are set to a temperature depending on the stabilizer used. As maximum temperature typically the melting point of the active ingredient is used, which is in the present case at about 150°C to about 160°C for solifenacin succinate. The screws in the extruder transport the material through the different zones which also comprise mixing elements, kneating elements especially for mechanical addition of energy as well as elements for applying pressure. The speed of the screws and the optimal pressure at the nozzle is optimized in accordance with the stabilizer used, e.g. the rotation speed of the screws can be set to about 150 rpm. At the exit (nozzle) of the extruder, the melted mass is pressed through small holes, e.g. those having a diameter of about 1 mm. Thereby, strings of the extrudate can be obtained, which solidify and cool when exiting the holes. The strings of extrudate are preferably broken up afterwards, e.g. by grinding.

If grinding is used as method to obtain the stabilized amorphous solifenacin or the pharmaceutically acceptable salt thereof, the above described ratios of stabilizer to solifenacin or the pharmaceutically acceptable salt thereof are preferably used, in particular about 6 to about 8 parts of the stabilizer, which is preferably PVP-VA, MC or mannitol to about 1 part per weight of solifenacin. The stabilizer and solifenacin or the pharmaceutically acceptable salt thereof are mixed together and placed into a suitable high energy mill, such as a high energy planetary mill, preferably at ambient temperature and under inert gas, such as dry nitrogen atmosphere. Within a high energy planetary mill typically grinding balls, such as ZrO₂ balls, having a diameter of e.g. 15 mm, are added and the mixture is intensively grinded, such as for a time of about 10 hours at 4,000 rpm.

The stabilized amorphous solifenacin or the pharmaceutically acceptable salt thereof obtained by the above described methods of spray drying, melt extruding or grinding typically shows a halo picture in the XRD, indicating that only amorphous solifenacin is present, and the glass transition point increased by about 30°C to about 60°C to result in a glass transition temperature of about 60°C to about 120°C with respect to unstabilized solifenacin (DSC measurement). This indicates that the amorphous solifenacin is stabilized by covering with the stabilizer.

In one preferred embodiment of the present application, the amorphous solifenacin or the pharmaceutically acceptable salt thereof, which is preferably stabilized solifenacin and which is more preferably covered by a stabilizer, is contained in the pharmaceutical composition of the present application in the form of particles having a particle size of about 10 µm to about 200 µm, more preferably of about 10 µm to about 100 µm.

The pharmaceutical composition of the present application comprises solifenacin or a pharmaceutically acceptable salt thereof which is preferably in stabilized form, more preferably covered by a stabilizer, preferably in an amount up to about 30 wt %, more preferably up to about 20 wt %, even more preferably in a range of about 2 to about 15 wt %, in particular in a range of about 5 to about 10 wt %, such as about 8 wt %, based on the total weight of the composition. Correspondingly, the pharmaceutical composition of the present application comprises stabilizer in the amounts that the ratio of stabilizer to solifenacin is such as described above, based on parts by weight and on the total weight of the composition.

The pharmaceutical composition of the present application may further comprise suitable pharmaceutically acceptable excipients and adjuvants, such as diluents, disintegrants, binders, glidants, lubricants, as well as coloring and sweetening agents. If the pharmaceutical composition of the present application is intended to be subjected to direct compression to obtain a solid pharmaceutical composition of the present application, the excipients and adjuvants have to be chosen such that these are suitable for direct compression. Which excipients and adjuvants are suitable for direct compression can be determined by standard methods and is known to the person skilled in the art.

As diluents for the pharmaceutical composition of the present application, sugars like mannitol, lactose, preferably lactose monohydrate, starch and its derivatives, and celluloses and its derivatives, in particular microcrystalline cellulose, low-substituted hydroxypropylcellulose (L-HPC) can be exemplified. The pharmaceutical composition of the present application can contain about 0 to about 80 wt % of a diluent, preferably about 10 to about 60 wt % of a diluent, in particular about 20 to about 45 wt % of a diluent, such as about 30 to about 42 wt % of a diluent, based on the total weight of the composition.

As disintegrant suitable for the pharmaceutical composition of the present application, starches, modified starches such as sodium starch glycolate, corn starch, pregelatinized starch, celluloses such as microcrystalline celluloses, carboxymethylcelluloses such as modified sodium carboxymethylcellulose, e.g. croscarmellose, crospovidone and mixtures thereof can be exemplified. The pharmaceutical composition of the present invention preferably can contain about 0 to about 20 wt % of a disintegrant, more preferably about 2 to about 15 wt % of a disintegrant, in particular about 3 to about 10 wt % of a disintegrant, such as about 4 to about 5 wt % of a disintegrant, based on the total weight of the composition.

The pharmaceutical composition of the present invention may further comprise one or more suitable binders, for example povidone and/or lactose and its derivatives, such as lactose monohydrate. These binders are preferably present in an amount of about 0 to about 10 wt %, about 0.1 to about 5 wt %, in particular about 0.5 to about 2.5 wt %, based on the total weight of the composition.

The pharmaceutical composition of the present invention may further comprise one or more lubricants. Suitable lubricants are for example stearic acid and derivatives thereof, such as calcium stearate, natrium stearyl fumarate or magnesium stearate, which is preferred, as well as glycerol mono-, di- and tristearate, and saturated plant oil. The lubricant may be present in the pharmaceutical composition of the present invention in an amount of about 0 to about 2 wt %, preferably about 0.1 to about 1.5 wt %, such as about 1 wt %, based on the total weight of the composition.

As glidants to be used in the pharmaceutical composition of the present invention, talc and colloidal silicon dioxide can be exemplified. The pharmaceutical composition of the present invention preferably contains about 0 to about 2 wt % of a glidant, more preferably about 0.1 to about 1 wt % of a glidant, in particular about 0.3 wt % of a glidant, based on the total weight of the composition.

Preferably, the pharmaceutical composition of the present invention contains solifenacin, stabilizer, about 0 to about 80 wt %, in particular about 10 to about 50 wt % of a diluent, about 0 to about 20 wt %, in particular about 5 to about 15 wt % of a disintegrant, about 0 to about 10 wt % of a binder, about 0 to about 2 wt % of a glidant, and about 0 to about 2 wt % of a lubricant, each based on the total weight of the composition. The amounts of the ingredients of the pharmaceutical composition have to sum up to 100 wt %.

Besides the above compounds, the pharmaceutical composition of the present invention may further comprise various other conventional excipients and adjuvants as known to the person skilled in the art, such as coloring agents or sweeteners. However, preferably, the pharmaceutical composition of the present invention only contains the above mentioned excipients and adjuvants.

The present invention further relates to a solid pharmaceutical composition preferably obtainable by using the above described pharmaceutical composition and bringing it into a solid form. The solid pharmaceutical composition is preferably a tablet, in particular a tablet which is obtained by direct compression.

The present invention further relates to a process for the preparation of a pharmaceutical composition as described above comprising stabilized amorphous solifenacin or a pharmaceutically acceptable salt thereof, wherein the stabilized amorphous solifenacin is obtained by
a) spray drying of a solution or dispersion of the solifenacin or the pharmaceutically acceptable salt thereof and a stabilizer as defined above, or
b) melt extruding of a mixture of solifenacin or the pharmaceutically acceptable salt thereof and a stabilizer as defined above, or
c) grinding of a mixture of solifenacin or the pharmaceutically acceptable salt thereof and a stabilizer as defined above, and
d) optionally adding excipients and/or adjuvants to the stabilized amorphous solifenacin obtained in steps a), b) or c), and
e) optionally using the mixture obtained in steps a), b), c) or d) for the preparation of a solid pharmaceutical composition, preferably pressing the mixture obtained in steps a), b), c) or d) into a solid pharmaceutical composition, in particular a tablet.

Above step e) is preferably carried out by direct compression.

As the stabilized amorphous solifenacin is in particular stable as long as it is kept dry, steps wherein the stabilized amorphous solifenacin or the pharmaceutically acceptable salt thereof come into contact to an aqueous solvent, e.g. water, have to be avoided. Therefore, the pharmaceutical compositions of the present application comprising stabilized amorphous solifenacin or a pharmaceutically acceptable salt thereof are preferably subjected to direct compression, thereby avoiding steps like wet granulation, whereby a solid pharmaceutical composition of the present application is obtained, which is preferred. Devices and conditions how to conduct compression, in particular direct compression of a pharmaceutical composition is known to the person skilled in the art.

Preferably, the solid pharmaceutical composition of the present application, which are in particular tablets, have a weight of about 100 to about 600 mg and contain about 20 to about 300 mg of the amorphous solifenacin per tablet.

The present invention further relates to the use of stabilizers as defined above for the stabilisation of amorphous solifenacin in a pharmaceutical composition as described above.

The following examples are merely intended to illustrate the invention and should not be construed as limiting.

### Example 1

Preparation of stabilized amorphous solifenacin by spray drying:
30 g of Collidon 64 (PVP-VA) are solved in 665 g demineralised water under rigorous stirring, followed by addition of 5 g solifenacin succinate. The obtained solution is pumped by a peristaltic pump with 5 - 7 g/min into a spray dryer (Büchi B-191), and the obtained coprecipitate is separated by a cyclone. Parameters used: delivery air ventilator 90 %, delivery air temperature 130°C, exhaust air temperature 65 - 75°C, amount of air 0.7 liters/hour.

### Example 2

Preparation of stabilized amorphous solifenacin by spray drying:
The stabilized amorphous solifenacin was obtained as described in Example 1, except that 30 g methylcellulose (MC) was used instead of collidon.

### Example 3

Preparation of stabilized amorphous solifenacin by melt extrusion:
The stabilizer (PVP-VA, polymethacrylate, mannitol, maltitol, Eudragit E, Kollidon VA 64 or Isomalt) and solifenacin were mixed in a ratio of 5 parts : 1 part by weight in a drum mixer (300 g stabilizer + 50 g solifenacin succinate), and the resulting mixture was dosed into a double screw extruder (dosing rate: about 1 kg/hour).

The extruder consists of seven individually heatable cylinders, which are heated depending on the other parameters, such as materials used and dosing rate. The maximum temperature was set to slightly above the melting point of the active ingredient, i.e. solifenacin succinate, at 150 - 160°C. The screws of the double screw extruder transported the material through the different zones and comprised mixing elements, kneting elements or addition of mechanical energy as well as elements for applying pressure. The speed of the screws was about 150 rpm. At the exit (nozzle) of the extruder the melted material was pressed through eight holes with a diameter of 1 mm. The resulting extruded strings solidified and cooled after discharge and were converted into small particles afterwards in a grinder.

### Example 4

Preparation of stabilized amorphous solifenacin by grinding:
5 - 8 parts per weight of stabilizer (PVP-VA, MC or mannitol) and one part by weight of solifenacin succinate were mixed together and placed into a jar of a high energy planetary mill at ambient temperature and under dry nitrogen atmosphere (jar volume: 45cm³). 6 - 10 ZrO₂ balls (diameter: 15 mm) were added, and the mixture was grinded for at least 10 hours at 4,000 rpm.

The obtained powder showed a halo picture like XDR and the glass transition point shifted of about 40°C with respect to unstabilized solifenacin (DSC measurement), indicating amorphous stabilized solifenacin.

### Example 5

60 mg of coprecipitate, obtained by spray drying of 10 mg solifenacin succinate and 50 mg of HPMC as described in example 1 or 2 are mixed with 43.5 mg lactose monohydrate (diluent), 30 mg of corn starch (disintegrant) and 1.5 mg magnesium stearate (lubricant), followed by direct compression of said mixture into tablets.

### Example 6

60 mg of extrudate, obtained by melt extrusion of 10 mg solifenacin succinate and 50 mg of PVP-VA as described in example 3, are mixed with 43.5 mg lactose monohydrate (diluent),

30 mg of corn starch (disintegrant) and 1.5 mg magnesium stearate (lubricant), followed by direct compression of said mixture into tablets.

### Example 7

60 mg of coprecipitate, obtained by spray drying of 10 mg solifenacin succinate with 50 mg of PVP as described in example 1 or 2, were mixed with 60 mg microcrystalline cellulose (diluent), 14 mg of croscarmellose (disintegrant) and 1 mg of magnesium stearate (lubricant), followed by direct compression of said mixture into tablets.

### Example 8

60 mg of extrudate, obtained by melt extrusion of 10 mg solifenacin succinate with 50 mg of maltitol as described in example 3, were mixed with 60 mg microcrystalline cellulose (diluent), 14 mg of croscarmellose (disintegrant) and 1 mg of magnesium stearate (lubricant), followed by direct compression of said mixture into tablets.

### Example 9

60 mg of coprecipitate, obtained by spray drying of 10 mg solifenacin succinate with 50 mg of PVP-VA as described in example 1 or 2, were mixed with 43 mg L-HPC (low-substituted hydroxypropylcellulose; binder/diluent/disintegrant), 30 mg lactose monohydrate (diluent/binder), 0.5 mg colloidal silicium dioxide (glidant) and 1.5 mg natrium stearyl fumarate (lubricant), followed by direct compression of said mixture into tablets.

### Example 10

60 mg of extrudate, obtained by melt extrusion of 10 mg solifenacin succinate with 50 mg of Isomalt as described in example 3, were mixed with 43 mg L-HPC (low-substituted hydroxypropylcellulose; binder/diluent/disintegrant), 30 mg lactose monohydrate (diluent/binder), 0.5 mg colloidal silicium dioxide (glidant) and 1.5 mg natrium stearyl fumarate (lubricant), followed by direct compression of said mixture into tablets.

### Example 11

60 mg of coprecipitate, obtained by spray drying of 10 mg solifenacin succinate with 50 mg of MC as described in example 1 or 2, were mixed with 64.5 mg lactose monohydrate (binder/diluent), 2.5 mg povidone (binder), 6.0 mg crospovidone (disintegrant), 0.5 mg colloidal silicium dioxide (glidant) and 1.5 mg magnesium stearate (lubricant), followed by direct compression of said mixture into tablets.

### Example 12

60 mg of extrudate, obtained by melt extrusion of 10 mg solifenacin succinate with 50 mg of polymethyacrylate as described in example 3, were mixed with 64.5 mg lactose monohydrate (binder/diluent), 2.5 mg povidone (binder), 6.0 mg crospovidone (disintegrant), 0.5 mg colloidal silicium dioxide (glidant) and 1.5 mg magnesium stearate (lubricant), followed by direct compression of said mixture into tablets.

## Claims

1. Pharmaceutical composition comprising solifenacin or a pharmaceutically acceptable salt thereof, **characterized in that** the solifenacin or a pharmaceutically acceptable salt thereof is present in the composition substantially in amorphous form, the composition comprises a stabilizer and the ratio of the stabilizer to amorphous solifenacin or a pharmaceutically acceptable salt thereof based on parts per weight is at least about 2:1.

2. Pharmaceutical composition according to claim 1, wherein the amorphous solifenacin or a pharmaceutically acceptable salt thereof is stabilized by the stabilizer.

3. Pharmaceutical composition according to claims 1 or 2, wherein the solifenacin or a pharmaceutically acceptable salt thereof is stabilized by being covered by the stabilizer.

4. Pharmaceutical composition according to any of the preceding claims, wherein the stabilized solifenacin is **characterised by** an increase in glass transition temperature of about 30°C to about 60°C.

5. Pharmaceutical composition according to any of the preceding claims wherein the ratio of the stabilizer to amorphous solifenacin or the pharmaceutically acceptable salt thereof based on parts per weight is about 2:1 to about 20:1.

6. Pharmaceutical composition according to any of the preceding claims, wherein the ratio of the stabilizer to amorphous solifenacin or the pharmaceutically acceptable salt thereof based on parts per weight is about 5:1 to about 10:1.

7. Pharmaceutical composition according to any of the preceding claims, wherein the stabilizer is selected from the group consisting of PEG, HPMC, MC, PVP, PVP-VA, PVA, polymethacrylate, sorbitol, mannitol, maltitol, Eudragit E, Kollidon VA 64 and Isomalt.

8. Pharmaceutical composition according to any of the preceding claims, wherein the amorphous solifenacin or a pharmaceutically acceptable salt thereof is present in form of particles having a particle size of about 10 µm to about 200 µm.

9. Pharmaceutical composition according to any of the preceding claims, **characterized in that** the composition comprises one or more excipients or adjuvants, selected from the group consisting of diluents, disintegrants, binders, glidants and lubricants.

10. Pharmaceutical composition according to any of the preceding claims, **characterized in that** it further comprises
about 0 to about 80 wt % of a diluent,
about 0 to about 20 wt % of a disintegrant,
about 0 to about 10 wt % of a binder,
about 0 to about 2 wt % of a glidant and
about 0 to about 2 wt % of a lubricant,
each based on the total weight of the composition.

11. Pharmaceutical composition according to any of the preceding claims, which is in form of a solid pharmaceutical composition, in particular a tablet.

12. Solid pharmaceutical composition according to claim 11, obtainable by direct compression.

13. Process for the preparation of a pharmaceutical composition as defined in any of the preceding claims comprising
a) spray drying of a solution or dispersion of solifenacin or a pharmaceutically acceptable salt thereof and a stabilizer, or
b) melt extruding of a mixture of solifenacin or a pharmaceutically acceptable salt thereof and a stabilizer, or
c) grinding of a mixture of solifenacin or a pharmaceutically acceptable salt thereof and a stabilizer, and
d) optionally adding excipients and/or adjuvants to the mixture obtained in steps a), b) or c) and
e) optionally using the mixture obtained in steps a), b), c) or d) for the preparation of a solid pharmaceutical composition.

14. Process according to claim 13, wherein the solid pharmaceutical composition of step e) is a tablet.

15. Use of a stabilizer selected from the group consisting of PEG, HPMC, MC, PVP, PVP-VA, PVA, polymethacrylate, sorbitol, mannitol, maltitol, Eudragit E, Kollidon VA 64 and Isomalt for the stabilisation of amorphous solifenacin or a pharmaceutically acceptable salt thereof.
